# EUROPEAN PATENT SPECIFICATION

(11) **EP 3 765 844 B1**
(45) Date of publication and mention of the grant of the patent: **07.06.2023**
(21) Application number: 19711435.8
(22) Date of filing: 12.03.2019
(51) Int. Cl.: G01N 33/487, G01N 33/03, G01N 33/14, G01N 33/49, G01N 31/22, G01N 21/78, G01N 33/493

(54) **METHOD FOR DETERMINING THE ANTIOXIDANT CAPACITY OF A BIOLOGICAL SAMPLE**
VERFAHREN ZUR BESTIMMUNG DER ANTIOXIDATIVEN KAPAZITÄT EINER BIOLOGISCHEN PROBE
PROCÉDÉ DE DÉTERMINATION DE CAPACITÉ ANTIOXYDANTE D'UN ÉCHANTILLON BIOLOGIQUE

(30) Priority: 13.03.2018 IT 201800003475
(43) Date of publication of application: 20.01.2021
(73) Proprietor: Fondazione Istituto Italiano di Tecnologia, 16163 Genova (IT)
(72) Inventor: PEDONE, Deborah, 16163 Genova (IT); MOGLIANETTI, Mauro, 16163 Genova (IT); POMPA, Pier Paolo, 16163 Genova (IT)
(74) Representative: Comoglio, Elena
(86) International application number: PCT/IB2019/051970
(87) International publication number: WO 2019/175749

(56) References cited:
- WO-A1-2007/039775
- US-A1- 2014 162 368
- US-A1- 2017 199 179
- MUSTAFA ÖZYÜREK ET AL: "Development of a Silver Nanoparticle-Based Method for the Antioxidant Capacity Measurement of Polyphenols", ANALYTICAL CHEMISTRY, vol. 84, no. 18, 27 August 2012 (2012-08-27), pages 8052-8059, XP055530929, US ISSN: 0003-2700, DOI: 10.1021/ac301925b

## Description

The present invention relates to a method for determining the antioxidant capacity of a biological sample.

The oxidative stress and the power of modulating it through antioxidants have attracted much attention in the scientific community, since numerous scientific studies and epidemiological investigations have linked oxidative stress to various diseases such as cancer, certain neurodegenerative diseases such as Alzheimer disease, and male infertility. In this context, saliva and other biological fluids such as blood, sweat and urine have important diagnostic potential, reflecting the physiological state of the individual at the time of sample collection. In particular, saliva is known to contain molecules produced by the salivary glands together with serum components transported into the saliva by passive diffusion through the capillaries, as well as other material released by the cells.

To date, there is a large body of literature linking the antioxidative state of saliva and other biological fluids to the onset of serious pathologies. The oxidative stress values in saliva and blood have also been shown to be strongly correlated.

The scientific and medical community is therefore focusing on various salivary biomarkers that allow monitoring of the body condition, such as for example the Total Antioxidant Capacity (TAC). TAC is of great interest because it comprises all the components capable of modulating oxidation, including enzyme and non-enzyme molecules, thus representing the net antioxidant power. TAC modifications can be bidirectional and denote systemic or specific changes in the redox homeostasis of certain tissues.

At the same time, there is a growing interest in the role that antioxidants in food play in human health, given that the beneficial effect resulting from the consumption of fruit, vegetables, tea, coffee and cocoa has actually been attributed to antioxidants.

To date, the main methods for determining the antioxidant capacity of a sample of a biological fluid or a food, for clinical or food analysis, are based on the power of an antioxidant to reduce the free radicals generated through a synthetic procedure, reduce specific metal ions (for example, copper, gold, iron), block fluorogenic radical molecules or modify chromogenic substrates (for example: ABTS (2,2'-azino-bis(3-ethylbenzothiazoline-6-sulphonic acid), DPPH (1,1-diphenyl-2-picrylhydrazyl), TMB (3,3',5,5'-tetramethylbenzidine), or block superoxide anions synthetically produced.

Much attention has been focused on the development of new tests capable of measuring the total antioxidant power of a given biological sample, in order to be able to assess the total antioxidant capacity thereof. The excessive variety of the available tests, however, has produced a lack of consistency among the results obtainable with the different systems, with a consequent lack of homogeneity and fragmentation of the results. Often, it is also necessary to resort to the use of multiple tests to assess the overall real antioxidant power of a sample.

There is also a lack of tests that lend themselves to be implemented through portable devices, point-of-care devices, and devices executable by the end user. A test with these characteristics would allow a continuous and effective monitoring of the antioxidant power of the organism through the main biological fluids.

An ideal standard method should possess essentially all of the following characteristics:
- measure the sample directly, without purification steps;
- be simple and low cost;
- have a clear physical-chemical mechanism of operation;
- not require specific instrumentation;
- be reproducible and reliable;
- be stable in ambient conditions;
- measure both lipophilic and hydrophilic antioxidants;
- be usable for studies on large numbers of samples.

The methods developed so far fall into two main categories: Hydrogen Atom Transfer (HAT) and Single Electron Transfer (SET). Methods belonging to the first category measure the ability of an antioxidant to block the free radicals through the donation of hydrogen. Instead, SET methods measure the ability of an antioxidant to transfer an electron to reduce a compound, whether it is a metal, a carbonyl or a radical.

The most common HAT methods are:
- Total radical-trapping antioxidant parameter (TRAP)
- Oxygen radical absorbance capacity
- Inhibition of induced LDL oxidation
- Total oxyradical scavenging capacity assay (TOSCA).

The most common SET methods are:
- Trolox equivalence antioxidant capacity (TEAC) assay
- Ferric ion reducing antioxidant power (FRAP) assay
- Cupric ions reducing antioxidant power (CUPRAC)
- Total antioxidant potential assay with Cu-complex as oxidant
- 2,2-Diphenyl-1-picrylhydrazyl radical (DPPH) scavenging
- 2,2-Azinobis 3-ethylbenzthiazoline-6-sulphonic acid radical (ABTS) scavenging assay
- N,N-dimethyl-p-phenylenediamine radical (DMPD) scavenging assay

In addition to the two categories mentioned above, there are also methods that selectively measure the ability of a given substance to block certain biologically relevant oxidants. The most important of these are: Hydrogen peroxide scavenging assay, Superoxide anion radical scavenging assay, and Hydroxyl radical scavenging assay.

New methods for determining the antioxidant power of fluids have been emerging in recent years and consist in the use of gold nanoparticles as a colorimetric sensor. These methods are based on the reduction of gold ions into nanoparticles with the consequent generation of colour associated with the formation of metal nanoparticles. The property of antioxidants to increase the size of the gold nanoparticles and hence their optical properties has also been proposed as a method of detecting antioxidants.

Another method uses the inhibition of the formation of gold nanoparticles by hydrogen peroxide, a phenomenon that results in a lack of staining of the solution. A method also based on gold nanoparticles and their optical properties has further been proposed for detecting antioxidants, which is based on the clustering of colloidal gold nanoparticles and the consequent colour change of the solution.

A further known method for detecting antioxidants in food uses cerium nanoparticles and is based on the change in colour of the nanomaterial upon contact with the food in the liquid phase.

WO2007/039775 discloses a method for measuring the total antioxidant capacity of a body fluid sample or foodstuffs and oils by measuring the decolourization of TMB spectrophotometrically al 450 nm in combination with an oxidizing agent (HCl).

Some of the known methods for measuring the antioxidant power of a biological fluid or a food are based on redox reactions catalysed by antioxidant enzymes (e.g. peroxidase and catalase). However, the latter have high isolation and purification costs, and are extremely sensitive to proteases, pH and temperature. Therefore, the scientific interest is to develop "artificial enzymes", designated as nanozymes, including palladium nanoparticles, which are ideal for their efficient and selective catalase and peroxidase activities.

Palladium nanoparticles are obtained by simple and inexpensive synthesis and purification protocols, are stable, maintain the catalytic activity, and remain unchanged even under extreme temperature and pH conditions, and resist the action of proteases. They are also able to oxidise chromogenic peroxidase substrates (in particular 3,3',5,5'-tetramethylbenzidine (TMB), 3,3'-diaminobenzidine (DAB)) in the presence of hydrogen peroxide as the oxidizing agent, with a higher affinity for TMB than biological peroxidase.

In the literature, there are few examples of colorimetric tests based on the use of palladium nanoparticles for the detection of biomarkers.

One of the first biological assays based on the use of palladium nanoparticles was proposed by Jianming Lan, et al., "Colorimetric determination of sarcosine in urine samples of prostatic carcinoma by mimic enzyme palladium nanoparticles"; Analytica Chimica Acta, Volume 825, 2014, doi.org/10.1016/j.aca.2014.03.040, who developed a sarcosine detection system based on the detection by means of TMB and palladium nanoparticles of hydrogen peroxide produced by the enzyme sarcosine oxidase upon sarcosine degradation.

Nanoparticles consisting of palladium and iron deposited on three-dimensional structures of graphene were instead proposed as substitutes to peroxidase in an assay for the colorimetric determination of glucose level in urine, based on the use of glucose oxidase, TMB and H₂O₂ (Xuejing Zheng, et al., "In-Situ Synthesis of Self-Assembled Three-Dimensional Graphene-Magnetic Palladium Nanohybrids with Dual-Enzyme Activity through One-Pot Strategy and Its Application in Glucose Probe"; ACS Applied Materials & Interfaces 2015 7 (6), 3480-3491, DOI: 10.1021/am508540x).

In the clinical field, instead, palladium nanoparticles functionalized with folic acid, associated with the TMB chromogen, have been used for the colorimetric detection of tumour cells, characterized by a higher expression of the folic acid receptor.

However, none of the currently available assays allows the antioxidant power to be assessed through a point-of-care, naked-eye determination, without the need for specific instrumentation.

These and other needs are met by the present invention, which provides a method for determining the antioxidant power of a sample of a biological fluid or a food which, compared to the techniques described in the state of the art, requires very short times (about 5 minutes), is based on the inhibition of colour development by antioxidants present in the sample itself, requires no pretreatment of the sample, and is extremely low cost. Moreover, the detection of the signal, i.e. the colour, can be performed with the naked eye and therefore on the spot ("point-of-care"), without necessarily requiring the use of specific instrumentation.

The method of the invention is as defined in appended claim 1.

Further features and advantages are defined in the dependent claims.

The claims form an integral part of the present specification.

The term "palladium nanoparticles" used in the present disclosure means that the nanoparticles do not contain metals other than palladium.

In the method of the present invention, the palladium nanoparticles perform a catalytic function. The reaction substrate is the chromogen TMB (3,3',5,5'-Tetramethylbenzidine). In the reaction, hydrogen peroxide is typically used as the oxidation agent. The substrate of the oxidation reaction is preferably the chromogen TMB (3,3',5,5'-tetramethylbenzidine). Hydrogen peroxide is typically used as the oxidizing agent.

The method of the invention is based on the fact that the antioxidant substances in the sample under examination interact with hydrogen peroxide, causing a partial or total inhibition of the TMB oxidation reaction. The result is a less intense development of the blue colour, which, as is known, is formed by oxidation of the TMB chromogenic substrate. It is important to note that the decreased oxidation of TMB by hydrogen peroxide is directly proportional to the amount of antioxidant substances and can therefore be used to colorimetrically quantify the concentration of antioxidants in the sample under examination. This reaction scheme is particularly effective and specific since it allows the total antioxidant capacity of a sample to be measured without the need for any preliminary purification step.

The assay method of the present invention can be applied to several biological fluids such as saliva, blood, sweat, or urine. It can also be applied to liquid food such as fruit juices and edible oils. In the case of oils, it is preferable that the sample is first mixed with a solution of methanol and isopropanol, to which the above listed reagents are subsequently added, without however requiring separation or purification.

The examples that follow are provided for illustration purposes and make reference to the appended drawings, in which:
figure 1 shows the results of a test performed by UV-visible spectroscopy on 15 samples of saliva obtained from healthy volunteers;
figure 2 shows a representative image of the results of the colorimetric method to the naked eye according to the invention performed on 5 samples of saliva (the colour scale at the top is a guide for the interpretation of the test);
figure 3 shows the results of a test carried out by UV-visible spectroscopy on food samples of fruit juice and other industrial beverages;
figure 4 shows a representative image of the results of the colorimetric method to the naked eye according to the invention performed on 5 samples of fruit juice (the colour scale at the top is a guide for the interpretation of the test).

The examples are provided purely by way of illustration and not of limitation of the scope of the invention as defined by the appended claims.

### EXAMPLES

The following assay is performed:
- 400 microlitres of an acetate buffer solution, typically between 0.01 and 1 M, preferably between 0.05 and 0.3 M were added to a test tube; the pH can vary between 1 and 7, preferably between 3 and 5.5;
- 200 microlitres of a TMB (3,3',5,5'-tetramethylbenzidine) solution were added at a concentration of between 0 and 1 M, preferably between 0.002 and 0.05 M;
- 100 microlitres of a solution containing palladium nanoparticles were added at a concentration comprised between 0.01 and 1000 ppm palladium, preferably between 0.1 and 10 ppm; the diameter of the nanoparticles may vary between 0.1 nm and 1000 nm, preferably between 1 and 100 nm;
- 18.3 microlitres of the sample to be tested were added;;
- the colour development reaction (blue) was initiated by the addition of 533 microlitres of a 1 M hydrogen peroxide solution.

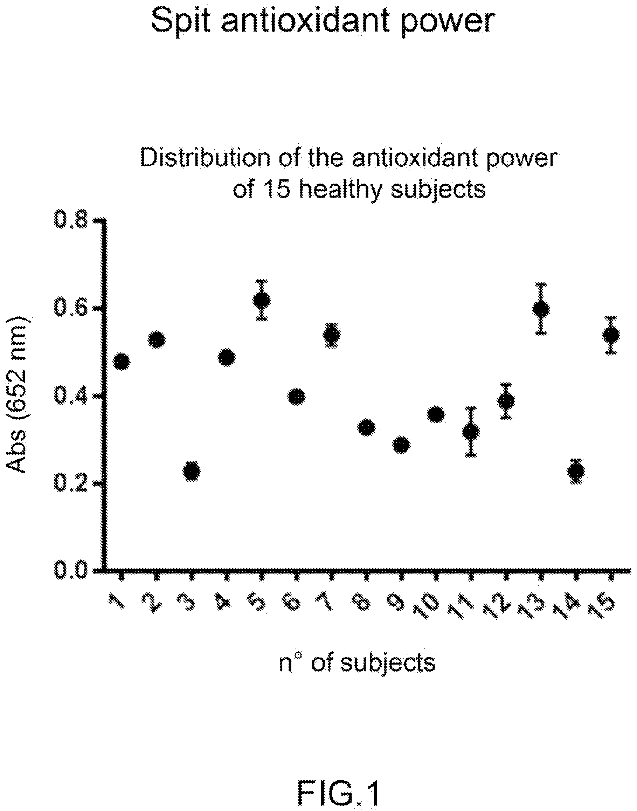

Figure 1 shows the results obtained by performing the test on 15 saliva samples obtained from healthy volunteers and carrying out the detection by UV-Vis spectroscopy (measurement of the absorbance at 652 nm).

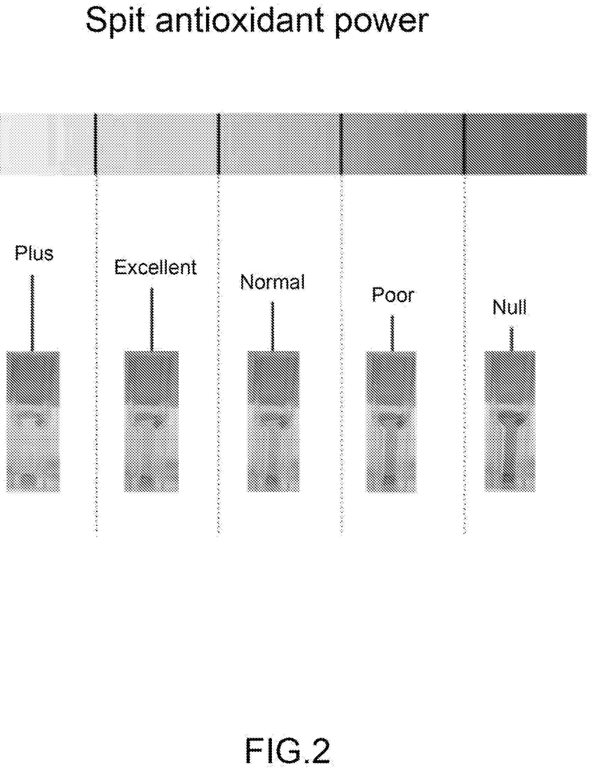

As an alternative to the spectroscopic method, it is possible to carry out the detection by the naked eye, using a reference colour scale which shows different colour intensity bands and the related antioxidant score (e.g., "excellent", "medium-high", "standard", "medium-low", "low", cf. Figure 2).

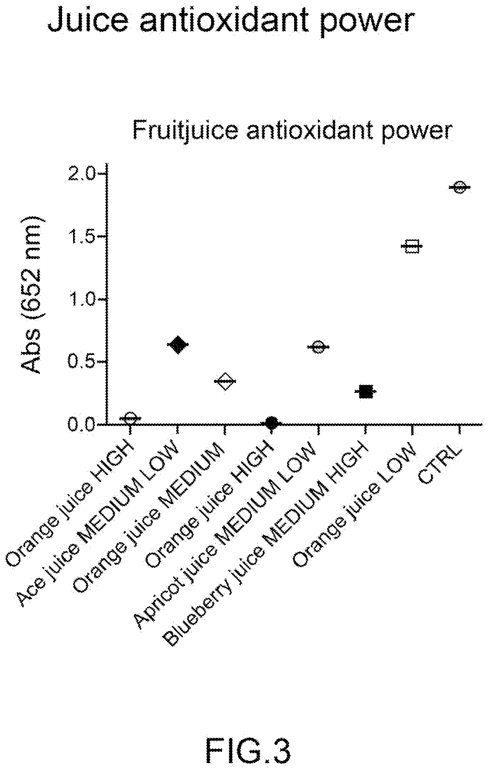

As indicated above, the method of the invention can also be used to analyse food samples such as fruit juices. Figure 3 shows the results obtained by testing food samples of fruit juice and other industrial beverages and performing the detection by UV-Vis spectroscopy (measurement of the absorbance at 652 nm).

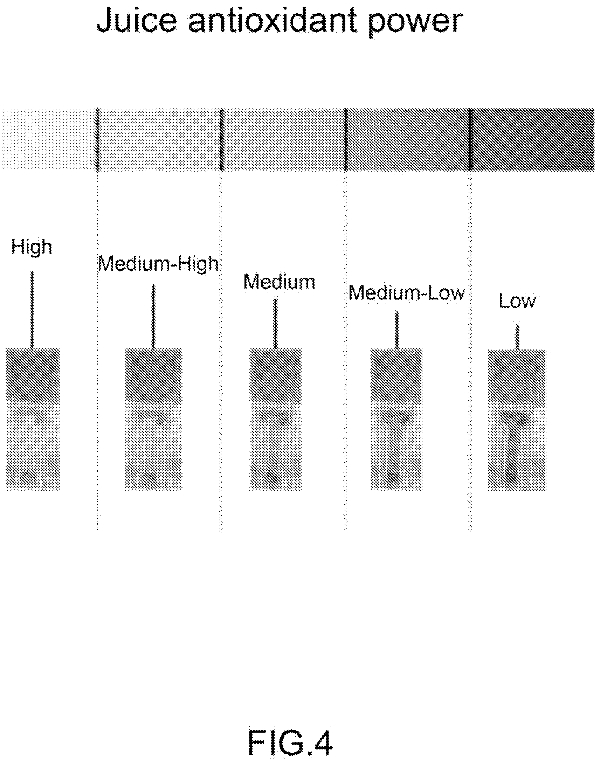

As an alternative to the spectroscopic method, it is possible to carry out the detection by the naked eye, using a reference colour scale which shows different colour intensity bands and the related antioxidant score (e.g., "excellent", "medium-high", "standard", "medium-low", "low", cf. Figure 4).

## Claims

1. A method for determining the antioxidant power of a sample of a biological fluid or a liquid food, comprising the steps of contacting the sample with an aqueous solution of an oxidising agent and a chromogenic peroxidase substrate, and detecting the colour intensity of the final solution thereby obtained, the colour intensity being proportional to the antioxidant power of the biological sample or liquid food sample, **characterized in that** the aqueous solution further comprises palladium nanoparticles.

2. The method according to claim 1, wherein the chromogenic peroxidase substrate is 3,3',5,5'-tetramethylbenzidine (TMB).

3. The method according to claim 1 or 2, wherein the oxidizing agent is hydrogen peroxide.

4. The method according to any one of claims 1 to 3, wherein the palladium nanoparticles have a diameter varying within the range of from 0.1 nm to 1000 nm, preferably of from 1 to 100 nm.

5. The method according to any one of claims 1 to 4, wherein the final solution is prepared in a buffer solution having a pH between 1 and 7, preferably between 3 and 5.5, the buffer solution being preferably an acetate buffer.

6. The method according to any one of claims 1 to 5, wherein the colour intensity of the final solution is detected with the naked eye by using a reference colour scale.

7. The method according to any one of claims 1 to 5, wherein the colour intensity of the final solution is detected by UV-visible spectroscopy.

8. The method according to claim 7, wherein the colour intensity of the final solution is detected by measuring the absorbance at a wavelength between about 600 and 700 nm, preferably around 650 nm, more preferably of 652 nm.

9. The method according to any one of claims 1 to 8, wherein the biological fluid is saliva, blood, sweat or urine.

10. The method according to any one of claims 1 to 8, wherein the liquid food is a fruit juice or an oil.

11. An *in vitro* diagnostic method for assessing the oxidative stress in a subject, comprising determining the antioxidant power of a biological fluid sample from the subject by the method according to any one of claims 1 to 9, wherein a decreased antioxidant power of the biological fluid sample from the subject compared to a reference sample or value is indicative of the oxidative stress of the subject.

12. The *in vitro* diagnostic method for assessing the oxidative stress in a subject according to claim 11, wherein the subject is suspected of conducting or conducts a health-damaging lifestyle, such as alcohol abuse or unhealthy diet, or the subject is suffering or is suspected to be suffering from a disease such as kidney damage, gout, endometriosis, diabetes or cancer.

## Patentansprüche

1. Verfahren zur Bestimmung des Antioxidationsvermögens einer Probe einer biologischen Flüssigkeit oder eines flüssigen Nahrungsmittels,
aufweisend die Schritte des Inkontaktbringens der Probe mit einer wässrigen Lösung eines Oxidationsmittels und eines chromogenen Peroxidasesubstrats und der Bestimmung der Farbintensität der dadurch erhaltenen finalen Lösung, wobei die Farbintensität proportional zu dem Antioxidationsvermögen der biologischen Probe oder der Probe des flüssigen Nahrungsmittels ist, **dadurch gekennzeichnet, dass** die wässrige Lösung außerdem Palladium-Nanopartikel aufweist.

2. Verfahren nach Anspruch 1, wobei das chromogene Peroxidasesubstrat 3,3',5, 5`-Tetramethylbenzin (TMB) ist.

3. Verfahren nach Anspruch 1 oder 2, wobei das Oxidationsmittel Wasserstoffperoxid ist.

4. Verfahren nach einem der Ansprüche 1 bis 3, wobei die Palladium-Nanopartikel einen Durchmesser haben, der im Bereich von 0,1 nm bis 1000 nm, bevorzugt von 1 bis 100 nm variiert.

5. Verfahren nach einem der Ansprüche 1 bis 4, wobei die finale Lösung in einer Pufferlösung mit einem pH-Wert zwischen 1 und 7, bevorzugt zwischen 3 und 5,5 hergestellt wird, wobei die Pufferlösung bevorzugt ein Acetat-Puffer ist.

6. Verfahren nach einem der Ansprüche 1 bis 5, wobei die Farbintensität der finalen Lösung unter Verwendung einer Referenzfarbskala mit bloßem Auge bestimmt wird.

7. Verfahren nach einem der Ansprüche 1 bis 5, wobei die Farbintensität der finalen Lösung durch UV-Spektoskopie bestimmt wird.

8. Verfahren nach Anspruch 7, wobei die Farbintensität der finalen Lösung durch Messung der Absorption bei einer Wellenlänge zwischen 600 und 700 nm, bevorzugt um 650 nm, weiter bevorzugt von 652 nm bestimmt wird.

9. Verfahren nach einem der Ansprüche 1 bis 8, wobei die biologische Flüssigkeit Speichel, Blut, Schweiß oder Urin ist.

10. Verfahren nach einem der Ansprüche 1 bis 8, wobei das flüssige Nahrungsmittel ein Fruchtsaft oder ein Öl ist.

11. *In vitro*-Diagnoseverfahren zur Bestimmung des oxidativen Stresses bei einer Versuchsperson, aufweisend die Bestimmung des Antioxidationsvermögens einer Probe einer biologischen Flüssigkeit der Versuchsperson durch das Verfahren nach einem der Ansprüche 1 bis 9, wobei ein verringertes Antioxidationsvermögen der Probe der biologischen Flüssigkeit der Versuchsperson im Vergleich mit einer Referenzprobe oder einem Referenzwert ein Indikator für den oxidativen Stress der Versuchsperson ist.

12. Das *In vitro*-Diagnoseverfahren zur Bestimmung des oxidativen Stresses bei einer Versuchsperson nach Anspruch 11, wobei die Versuchsperson vermutlich oder tatsächlich einen gesundheitsschädlichen Lebensstil führt, wie z.B. Alkoholmissbrauch oder ungesunde Ernährung, oder die Versuchsperson tatsächlich oder vermutlich an einer Krankheit wie Nierenschaden, Gicht, Endometriose, Diabetes oder Krebs leidet.

## Revendications

1. Procédé de détermination du pouvoir antioxydant d'un échantillon d'un fluide biologique ou d'un aliment liquide,
comprenant les étapes de mise en contact de l'échantillon avec une solution aqueuse d'un agent oxydant et d'un substrat de peroxydase chromogène, et de détection de l'intensité de couleur de la solution finale ainsi obtenue, l'intensité de couleur étant proportionnelle au pouvoir antioxydant de l'échantillon biologique ou de l'échantillon d'aliment liquide, **caractérisé en ce que** la solution aqueuse comprend en outre des nanoparticules de palladium.

2. Procédé selon la revendication 1, dans lequel le substrat de peroxydase chromogène est la 3,3',5,5'-tétraméthylbenzidine (TMB).

3. Procédé selon la revendication 1 ou 2, dans lequel l'agent oxydant est le peroxyde d'hydrogène.

4. Procédé selon l'une quelconque des revendications 1 à 3, dans lequel les nanoparticules de palladium ont un diamètre variant dans la plage de 0,1 nm à 1000 nm, de préférence de 1 à 100 nm.

5. Procédé selon l'une quelconque des revendications 1 à 4, dans lequel la solution finale est préparée dans une solution tampon ayant un pH entre 1 et 7, de préférence entre 3 et 5,5, la solution tampon étant de préférence un tampon acétate.

6. Procédé selon l'une quelconque des revendications 1 à 5, dans lequel l'intensité de couleur de la solution finale est détectée à l'oeil nu à l'aide d'une échelle chromatique de référence.

7. Procédé selon l'une quelconque des revendications 1 à 5, dans lequel l'intensité de couleur de la solution finale est détectée par spectroscopie UV-visible.

8. Procédé selon la revendication 7, dans lequel l'intensité de couleur de la solution finale est détectée par mesure de l'absorbance à une longueur d'onde entre environ 600 et 700 nm, de préférence d'environ 650 nm, de manière davantage préférée de 652 nm.

9. Procédé selon l'une quelconque des revendications 1 à 8, dans lequel le fluide biologique est de la salive, du sang, de la sueur ou de l'urine.

10. Procédé selon l'une quelconque des revendications 1 à 8, dans lequel l'aliment liquide est un jus de fruit ou une huile.

11. Procédé de diagnostic *in vitro* pour évaluer le stress oxydatif chez un sujet, comprenant la détermination du pouvoir antioxydant d'un échantillon de fluide biologique du sujet par le procédé selon l'une quelconque des revendications 1 à 9, dans lequel un pouvoir antioxydant réduit de l'échantillon de fluide biologique du sujet comparé à un échantillon ou valeur de référence est un indicateur de stress oxydatif chez un sujet.

12. Procédé de diagnostic in vitro pour évaluer le stress oxydatif chez un sujet selon la revendication 11, dans lequel le sujet est suspecté d'avoir ou a un mode de vie nocif pour sa santé, par exemple l'abus d'alcool ou un régime alimentaire malsain, ou le sujet souffre ou est suspecté de souffrir d'une maladie comme, par exemple, de lésion rénale, de goutte, d'endométriose, de diabète ou d'un cancer.
